# EUROPEAN PATENT SPECIFICATION

(11) **EP 0 385 552 B1**
(45) Date of publication and mention of the grant of the patent: **05.10.1994**
(21) Application number: 90200459.7
(22) Date of filing: 27.02.1990
(51) Int. Cl.: C12N 1/02

(54) **A process of flocculation-coagulation of fermentation broths of streptomyces sp. which permits direct obtainment of completely transparent liquids**
Verfahren zur Flokkulationskoagulation von Fermentationsbrühen von Streptomyces sp., wodurch direkt vollkommen transparente Flüssigkeiten enthalten werden können
Procédé de floculation-coagulation de bouillons de fermentation de streptomyces sp. pour obtenir directement des liquides complètement transparents

(30) Priority: 01.03.1989 ES 8900749
(43) Date of publication of application: 05.09.1990
(73) Proprietor: PHARMA MAR, S.A., 28046 Madrid (ES)
(72) Inventor: Garcia Alvarez, Ma. Concepcion, E-28002 Madrid (ES); De Urries Senante, Ma. Pilar J., E-28033 Madrid (ES); De La Calle Verdu, Fernando, E-28770 Colmenar Viejo, (Madrid) (ES); Diaz Del Aguila Sarda, Guillermo, Las Rozas, (Madrid) (ES)
(74) Representative: Ungria Lopez, Javier

(56) References cited:
- DD-A- 145 279
- DD-A- 215 332
- DD-A- 251 573
- DE-A- 2 159 028

## Description

### TECHNICAL FIELD OF THE INVENTION

The present invention refers to the use of the flocculation process in fermentation broths of Streptomyces sp.this physico-chemical process constituting an innovation in this technical field.

There is no prior art of flocculations of culture broths that permit a micelle separation simultaneous to deproteinization and elimination of metabolites that interfere in the subsequent process of purification isolation and identification of the corresponding active principle.

Spanish patent no. 537158 refers to the use of Praestol, as a filtration coadjuvant but no mention at all is made of the use of organic polyelectrolytes or of any other type of chemical substances as flocculating agents.

Up to the present, coagulation-flocculation has been a technique used, practically exclusively in treating waste water for the subsequent recovery and purification thereof.

The invention as claimed refers to a method of obtainment of flocculated fermentation broths, obtaining a simplification in the process of isolation of active principles, produced by different specifies of Streptomyces.

The clarification of the culture broth is done by means of the physico-chemical process of coagulation-flocculation. By choosing the suitable flocculant countertype, the colloidal material or material in suspension in the broth is eliminated, along with undesirable dissolved materials which make the purification process difficult.

According to this process, completely transparent broths are obtained without the need to use conventional methods such as centrifugation, adsorption on active carbon, filtration with coadjuvants, etc.

Likewise, larger yields are obtained upon reducing the number of steps prior to the extraction process itself to the maximum.

The flocculation of the smallest particles is started by adding inorganic electrolytes which disperse in the solution, favoring the intermolecular aggregation upon interacting with the surface of the particles and reducing the short range repulsion forces that delay or impede aggregation and sedimentation.

The increase of the ionic force of the fermentation broth favours the initial flocculation process.

Adding the high molecular weight organic polymers gives rise to the formation of larger and more compact flocculi, greatly facilitating their separation from the flocculated broth.

The fermentation broths of Streptomyces tend to be rather complex and the different metabolites of pharmacological interest that are produced can be found retained in the micelle, dissolved in the water, or part in the micelle and part in the aqueous solution. Processes such as cellular separation, deproteinization, desalination, etc. are necessary to effect the extraction of these compounds from the culture medium.

By means of vacuum rotary filtration or continuous centrifugation separation of cells from the broth can be attained but, later, it is necessary to effect desalination, deproteinization of the medium and elimination of the rest of the metabolites present in the culture broth, which would imply some very large losses of the active principle with regard to its initial concentration in the broth. The use of more sophisticated techniques in the process of extraction and purification, such as ultrafiltration and reverse osmosis, would not simplify the process since in order to use them it would be necessary to carry out preliminary filtrations on active carbon or ionic resins.

The present invention refers to a method of coagulation-flocculation which makes it possible to obtain completely transparent broths, with small losses, of the products of pharmacological interest which are to be extracted. On the other hand, the use of this process permits, when necessary, to effect simultaneous to the flocculation extraction with an organic solvent, as well as providing an aqueous solution clean enough to make its direct concentration possible, either by reverse osmosis or by ultrafiltration, depending on the molecular size of the compounds in question.

The inorganic coagulants that are used the most are hydrolyzable metal salts , such as aluminum sulfate, ferric sulfate and, more favourably, prehydrolyzed aluminum chlorides (aluminum polychlorides.)

The coagulation process is carried out advantageously if the ionic force of the medium is increased, for example, by adding an inorganic electrolyte to the fermentation broth.

The coadjuvant flocculation substances are organic poly-electrolytes, preferably synthetic organic polymers of the type of polyamines and polyacrylamides with a different degree of ionogeneity depending on the chemical nature of the active products to be extracted.

The object of the present invention is to provide a simple and efficient process for the obtainment of culture broths completely free of suspended cells, proteins, etc., which facilitates extraction of the active compounds as of fermentation broths of Streptomyces, once they have been flocculated and passed through rolling sieves (or subjected to simple filtration) for the separation of the flocculi formed. This method comprises the following steps:
a) Adding to the culture broth an inorganic electrolyte to favor the action of the inorganic coagulant, such as alkaline and alkaline earth metal chlorides, preferably sodium or potassium chlorides or, advantageously, ammonium chloride. Generally they are used in the proportion of 0.5-4 g/l of culture broth, however, this proportion may be higher depending on the culture mediums used.
b) Incorporating with stirring and at a pH of 5-8, preferably a pH of 6-7, an inorganic coagulant, preferably of the type of aluminum polychlorides with a content of Al₂O₃ of 10-20 %. The necessary amount varies between 2-20 ml/l of culture broth starting the flocculation of said broth and a reduction of the pH until a value between 3 and 4 is reached.
c) Once the flocculation process has started with the inorganic coagulant, the organic polyelectrolyte or a mixture thereof, generally polyamines or preferably synthetic polyacrylamides of a suitable ionic nature, is added. The pH required so that these flocculants have the maximum effect varies between 6-8 and the type of stirring as well as the duration thereof are a determining factor of the formation and size of the final flocculi. The solutions that are used of the different polyacrylamide countertypes are very diverse and the ones used the most are the ones with concentrations of 0.01-0.2 % and preferably ones of 0.05-0.1 %, with a total concentration in broth of 150-400 mg/l of culture broth.
d) Separating the flocculi from the fermentation broth which can be carried out by the use of rolling sieves or by simple filtration, followed by decantation of the phases in the cases in which the flocculation of the fermentation broth is effected in the presence of a water immiscible organic solvent, and once the completely transparent liquid is attained, if the case so requires, prior concentration of said transparent liquid, either by reverse osmosis or else by evaporation depending on whether the liquid is water or an organic solvent.

The method of coagulation-flocculation of fermentation broths of the present invention makes it possible to obtain completely transparent flocculated liquids in a simple, direct and efficient way. In the case of broths coming from Streptomyces clavuligerus yields of 85-95 % of the active substance with regard to the initial content thereof in the fermentation broth are obtained, thus permitting a big increase of the overall yields of obtainment of said active substances. In the examples that are specified in detail hereinafter, the advantages of the use of flocculating agents in the obtainment of completely transparent broths which permit the start of the extraction of the pharmacologically active metabolites are clearly put forth.

Some illustrative examples of the way of putting the present invention into practice are stated hereinafter.

### Example 1

An aqueous solution of an aluminum polyhydroxychloride, Prodefloc AR-180, in the proportion of 1/70, after adding an inorganic electrolyte, NH₄Cl, in the ratio of 1:1000 (w:v), is added to 250 ml. of Streptomyces clavuligerus fermentation broth (1650 mg/l of active compound.) The flocculation starts and increases as the aqueous solution is neutralized with NaOH. Keeping the stirring strong an organic polyelectrolyte, in this case Praestol 2540 at 0.05 %, in a concentration of 250 mg/l and water in the ratio of 1:5 (v:v) is added. Big flocculi which are then separated from the supernatant liquid appear and said liquid remains completely transparent and, as of it, the Z(2R,5R)-3-(2-hydroxyethyliden)-7-oxo-4-oxa-1-azabicyclo (3,2,0)-heptane-2-carboxylic acid can be directly extracted. The comparative yield of the active principle of the flocculated broth with regard to the cultivated one is 85 %.

### Example 2

1 liter of S. clavuligerus fermentation broth is flocculated. 1,2 g. of NH₄Cl and 13.5 ml/l of aluminum polychloride (Prodefloc AR-180) are added as a flocculation initiator. The formation of flocculi increases as the suspension is neutralized with NaOH. 300 mg. of Praestol 2395 in an 0.1 % aqueous solution are added and the previously formed flocculi are enlarged. 100 ml. of H₂O are added maintaining the stirring for some minutes. The separation of the flocculi leads to the obtainment of a completely transparent liquid which contains 95 % of active principle with regard to the culture broth.

### Example 3

A Streptomyces clavuligerus fermentation broth which has a content of 1025 mg/l of active principle is used.

500 ml. of this broth is flocculated with Prodefloc AR-100 aluminum polychloride, in the concentration of 30 ml/l. The NH₄Cl which is used as an inorganic electrolyte is added, in this case, in the ratio of 3:2000 (w:v.) The flocculi that are formed become considerably enlarged upon adding, after neutralization, Praestol 2395 in an 0.1 % aqueous solution and in the ratio of 1:5 (v:v.) After adding 50 ml/l. of water, the same is stirred vigorously for about 2 minutes and a disintegration of the flocculi formed previously is produced and it facilitates their separation. The yellow and completely transparent liquid which is obtained contains 902 mg/l of active principle referred to the fermentation broth.

### Example 4

500 ml. of Streptomyces clavuligerus fermentation broth (1730 mg/l) is flocculated with NH₄Cl and Prodefloc AR-180 in the ratio of 1:100 (v:v.) It is neutralized with NaOH and 85 mg. of Praestol 2500 in an 0.05 % aqueous solution are added as a flocculation coadjuvant. It is diluted with water and the flocculi are separated. The resulting liquid is completely transparent and contains 1478 mg/l of active compound with regard to the initial broth.

### Example 5

2 liters of a Streptomyces clavuligerus fermentation broth that contains 1100 µg/ml of active compound are flocculated. NH₄Cl in a ratio of 1:1000 (w/v) and Prodefloc AR-180 in a concentration of 10 ml/l as a flocculation initiator are used. It is neutralized with KOH and 480 ml. of an 0.1 % aqueous solution of Praestol 2395 are added. When the flocculi start becoming enlarged 200 ml. of water are added. The stirring is maintained for a few minutes for the total formation of these flocculi and then they are separated by means of rolling sieves. The transparent liquid that is obtained has its volume reduced by means of the process of reverse osmosis. The equivalent concentration in the concentrated broth is 86 % with regard to the fermentation broth. As of this concentrate the active principle is extracted with organic solvents.

### Example 6

Similar to example 3 but using Crosefloc A-200 in an 0.1 % aqueous solution and concentration of 200 mg/l as the organic polyelectrolyte.

### Example 7

A S. clavuligerus fermentation broth (500 ml.) is flocculated with NH₄Cl (1.2 g/l) and Prodefloc AR-180 in the ratio of 1:80 (v:v.) Crosefloc A-304 in an 0.1 % aqueous solution and in the amount of 280 mg/l is added and the pH has been previously brought to 7 with KOH. It is diluted with H₂O and after a few minutes of stirring compact flocculi which are rapidly separated from the completely transparent supernatant liquid are formed. The yield with which the active principle is obtained is 85 %.

### Example 8

One proceeds in this case as in example 3 introducing the use of the same concentration of Praestol 2395 but in an 0.2 % aqueous solution as a variant. The yield is similar to the one obtained in the case of example 3.

### Example 9

A liter of S. clavuligerus culture broth with a content of Z(2R,5R)-3-(2-hydroxyethyliden)-7-oxo-4-oxa-1-azabicyclo (3,2,0)-heptane-2-carboxylic acid of 950 mg/l. is flocculated with 1.6 g. of NH₄Cl and 12 ml. of Prodefloc AR-180 (the pH drops to 3.9), NaOH is added until a pH of 7 is obtained and 200 ml. of an 0.1 % synthetic polyacrylamide solution, in this case Magnafloc 156, are added. The flocculi formed disintegrate with vigorous stirring facilitating the separation of the same and a yellow transparent liquid with a concentration of active principle of 4.2 mmol/l referred to the culture broth remains. This flocculated liquid is concentrated by means of the process of reverse osmosis in order to proceed with the extraction of the pharmacologically active product.

### Example 10

250 ml. of a S. clavuligerus fermentation broth with a content of active principle of 2090 µg/ml are flocculated with NH₄Cl (1 g/l) and Prodefloc AR-180 (14 ml/l) neutralizing with NaOH. The flocculi become larger by adding 70 ml. of 0.1 % Praestol 2395 and 50 ml. of water. The separation of the flocculi permits the obtainment of a completely transparent liquid. The yield o± active principle with regard to the cultivated broth is 86.4 %.

### Example 11

Two liters of a Streptomyces clavuligerus fermentation broth are flocculated with 2 g. of ammonium chloride and 28 ml. of Prodefloc AR-180 as a coagulating agent. 560 mg. of the flocculation coadjuvant (Praestol 2395 in an 0.1 % aqueous solution) are added to the previously neutralized suspension and then it is diluted with water and the stirring is maintained for a few minutes in order to facilitate the disintegration and separation of the flocculi. The yield is 92 %.

### Example 12

500 ml. of S. clavuligerus fermentation broth are flocculated with 0.7 g. of NH₄Cl, 7 ml. of Prodefloc AR-180 (neutralization with KOH) and 0.1 % Praestol 2540 at a concentration of 280 ml:l. Starting from a concentration of active principle of 1340 mg/l. a concentration of 1165.6 mg/l. referred to the initial broth is reached.

### Example 13

500 ml. of a S. clavuligerus culture broth (1275 mg/l of active substance) are flocculated using, once the ammonium chloride has been added, 14 ml/l of Prodefloc AR-180 as a coagulating agent. Once the resulting suspension has been neutralized with NaOH, 120 mg. of flocculation coadjuvant, in this case Magnafloc 919 in an 0.05 % aqueous solution and water in a ratio of 1:5 (v:v), are added. After the separation of the flocculi formed, a completely transparent liquid which contains 5.5 mmol/l of fermentation broth is obtained.

### Example 14

An aqueous solution of an aluminum polyhydroxychloride, Prodefloc AR-180, in the proportion of 7:500 after adding 1.5 g/l of NH₄Cl, is added to 250 ml. of a Streptomyces clavuligerus cultivation broth. It is neutralized with NaOH and 70 mg. of Praestol 2395 in an 0.1 % aqueous solution are added. Water is added until a volume of 400 ml. is attained and the stirring is kept for a few minutes. The flocculi formed are allowed to settle for their subsequent separation. The yield with which the Z(2R,5R)-3-(2-hydroxyethyliden)-7-oxo-4-oxa-1-azabicyclo(3,2,0)-heptane-2-carboxylic acid is obtained is 94 %.

## Claims

1. A process of flocculation-coagulation of fermentation broths of Streptomyces sp. which permits the direct obtainment of completely transparent liquids, facilitating the simultaneous separation of cells, proteins, salts and metabolites which interfere in the process of isolation of the active principles, characterized in that it comprises the following steps:
a) adding an inorganic electrolyte to the culture broth to enhace the action of the coagulant;
b) incorporating an inorganic coagulant as an initiator of the coagulation process with stirring and at a pH of 5-8;
c) adding, once the flocculation has started, an organic polyelectrolyte, with stirring and keeping the pH at 6-8;
d) separating the flocculi formed from the fermentation broth, using rolling sieves or simply filtration; optionally, if the flocculation has been carried out in the presence of a water immiscible organic solvent, decantation of the phases, once the flocculi are formed and separated; and, if necessary, concentration of the liquid by reverse osmosis or evaporation.

2. A process according to claim 1, characterized in that the inorganic electrolyte used in step (a) is selected from among alkaline and alkaline earth metal chlorides and ammonium chloride.

3. A process according to claim 2, characterized in that the chloride is sodium chloride or potassium chloride.

4. A process according to claim 1, characterized in that the inorganic coagulant used in step (b) is an aluminum polychloride with a content of Al₂O₃ of 10-20%.

5. A process according to claim 1, characterized in that the organic polyelectrolyte used in step (c) is a synthetic polyacrylamide or polyamine.

## Patentansprüche

1. Verfahren zur Flokkulations-Koagulation von Fermentationsbrühen von Steptomyces sp., das den direkten Erhalt vollständig transparenter Flüssigkeiten erlaubt, wobei die gleichzeitige Abtrennung von Zellen, Proteinen, Salzen und Metaboliten erfolgt, die im Isolierungsverfahren des Wirkstoffes stören, dadurch **gekennzeichnet,** daß es die folgenden Schritte umfaßt:
a) Zugabe eines anorganischen Elektrolyten zur Kulturbrühe unter Wirkungsverstärkung des Koagulans;
b) Zugabe eines anorganischen Koagulans als Starter für das Koagulationsverfahren unter Rühren und bei einem pH von 5 bis 8;
c) Zugabe, nachdem die Flokkulation eingesetzt hat, eines organischen Polyelektrolyten unter Rühren und Einhaltung eines pH-Wertes bei 6 bis 8;
d) Abtrennung der gebildeten Flokkuli aus der Fermentationsbrühe unter Verwendung von Rollsieben oder einfacher Filtration, gegebenenfalls, falls die Flokkulation in Gegenwart von einem in Wasser nicht mischbaren organischen Lösungsmittel durchgeführt wurde, Dekantieren der Phasen, nachdem die Flokkuli gebildet und abgetrennt sind, und falls notwendig, Konzentrierung der Flüssigkeit durch reverse Osmose oder Verdampfung.

2. Verfahren nach Anspruch 1, dadurch **gekennzeichnet,** daß der in Schritt a) verwendete anorganische Elektrolyt ausgewählt ist aus Alkalimetall- und Erdalkalimetallchloriden und Ammoniumchlorid.

3. Verfahren nach Anspruch 1, dadurch **gekennzeichnet,** daß das Chlorid Natriumchlorid oder Kaliumchlorid ist.

4. Verfahren nach Anspruch 1, dadurch **gekennzeichnet,** daß das in Schritt b) verwendete anorganische Koagulans ein Aluminiumpolychlorid mit einem Gehalt von Al₂O₃ von 10 bis 20 % ist.

5. Verfahren nach Anspruch 1, dadurch **gekennzeichnet,** daß der in Schritt c) verwendete organische Polyelektrolyt ein synthetisches Polyacrylamid oder Polyamin ist.

## Revendications

1. Procédé de floculation-coagulation de bouillons de fermentation de Streptomyces sp. qui permet l'obtention directe de liquides complètement transparents, facilitant la séparation simultanée de cellules, protéines, sels et métabolites qui interfèrent dans le processus d'isolement des principes actifs, caractérisé en ce qu'il comprend les étapes suivantes :
a) addition d'un électrolyte inorganique au bouillon de culture pour améliorer l'action du coagulant ;
b) incorporation d'un coagulant inorganique comme initiateur du processus de coagulation sous agitation et à un pH de 5-8 ;
c) addition, une fois la floculation commencée, d'un polyélectrolyte inorganique, sous agitation et en maintenant le pH à 6-8 ;
d) séparation des flocons formés à partir du bouillon de fermentation, en utilisant des tamis rotatifs ou simplement par filtration ; éventuellement, si la floculation a été effectuée en présence d'un solvant organique non miscible dans l'eau, décantation des phases, une fois les flocons formés et séparés ; et, si nécessaire, concentration du liquide par osmose inverse ou évaporation.

2. Procédé selon la revendication 1, caractérisé en ce que l'électrolyte inorganique utilisé dans l'étape (a) est choisi parmi les chlorures de métaux alcalins et alcalino-terreux et le chlorure d'ammonium.

3. Procédé selon la revendication 2, caractérisé en ce que le chlorure est du chlorure de sodium ou du chlorure de potassium.

4. Procédé selon la revendication 1, caractérisé en ce que le coagulant inorganique utilisé dans l'étape (b) est un polychlorure d'aluminium avec une teneur en Al₂O₃ de 10-20 %.

5. Procédé selon la revendication 1, caractérisé en ce que le polyélectrolyte organique utilisé dans l'étape (c) est un polyacrylamide ou une polyamine synthétique.
